# EUROPEAN PATENT APPLICATION

(11) **EP 4 830 629 A1**
(43) Date of publication of application: **28.10.2026**
(21) Application number: 24818475.6
(22) Date of filing: 17.05.2024
(51) Int. Cl.: A61B 5/245

(54) **ATOMIC MAGNETOMETER MAGNETOENCEPHALOGRAPHY SYSTEM BASED ON WIRELESS TRANSMISSION, AND MAGNETOENCEPHALOGRAPHY DETECTION METHOD**

(30) Priority: 30.01.2024 CN 202410127475
(71) Applicant: Changping National Laboratory, Beijing 102206 (CN); Peking University, Beijing 100871 (CN)
(72) Inventor: GAO, Jiahong, Beijing 102206 (CN); HE, Kaiyan, Beijing 102206 (CN); CHENG, Hao, Beijing 102206 (CN); YIN, Yugang, Beijing 102206 (CN); PANG, Wei, Beijing 102206 (CN)
(74) Representative: Metroconsult Srl
(86) International application number: PCT/CN2024/093879
(87) International publication number: WO 2024/250942

(57) **Abstract**

The present disclosure provides a wireless transmission-based optically pumped magnetometer-magnetoencephalography (OPM-MEG) system and a MEG detection method, which relate to the technical field of MEG detection. The wireless transmission-based OPM-MEG system includes a wearable structural member (100), an OPM (200), an acquisition host computer (300), and a control module (400). The OPM (200) is configured to form a magnetic field detection array, and the acquisition host computer (300) is configured to store the MEG signals acquired by the OPM (200). The control module (400) is electrically connected to the OPM (200) via a flexible printed circuit (700), and the control module (400) is connected to the acquisition host computer (300) via wireless communication. The control module of the present disclosure does not need to be connected to the acquisition host computer via wired circuits, so that a subject is free from the restraints of wired circuits during measurement, which solves the problem of limited movement of the subject. No wired circuits are required between the control module and the acquisition host computer, eliminating the need to consider specifications and costs of wired circuits.

## Description

### RELATED APPLICATION

The present disclosure claims priority to Chinese Invention Patent Application No. 202410127475.0, filed January 30, 2024, and entitled "wireless transmission-based optically pumped magnetometer-magnetoencephalography system and magnetoencephalography detection method".

### TECHNICAL FIELD

The present disclosure relates to the field of magnetoencephalography technology, and in particular, to a wireless transmission-based optically pumped magnetometer-magnetoencephalography (OPM-MEG) system and a magnetoencephalography detection method.

### BACKGROUND

Magnetoencephalography (MEG), as a non-invasive functional imaging technique for real-time detection of post-synaptic magnetic changes in cerebral neurons, boasts wide application values and broad development prospects in clinical medicine and neuroscience research. Magnetic field detection technologies for MEG fall into two categories: Superconducting Quantum Interference Device (SQUID) and Optically Pumped Magnetometer (OPM).

SQUID-MEG operates in a superconducting environment, requiring a Dewar vessel to store liquid helium and provide thermal insulation. This results in an overall bulky and immobile system, severely restricting subject movement and greatly limiting experimental paradigm design. Moreover, due to thermal insulation requirements, the magnetic detection sensor units are located far from the scalp, leading to severe attenuation of the collected MEG signals.

In contrast, OPM-MEG operates at ambient temperature, featuring superior ultimate sensitivity, flexible array layout and improved spatiotemporal resolution. It demonstrates better performance and application prospects compared to SQUID-MEG, enabling MEG signal detection at positions much closer to the scalp, thus providing greater sensitivity to MEG signals - a factor particularly important in neuroscience research focused on weak MEG signals. In addition, OPM-MEG is compact and lightweight, offering greater convenience for use in diverse scanning environments. Therefore, the lightweight design and strong environmental adaptability of OPM-MEG greatly expand the application scope of MEG detection technology, bringing more possibilities to brain science research and clinical practice.

In the prior art, an OPM-MEG system generally includes a magnetic shielding apparatus, an OPM detector array, an OPM control system, a host computer, and supporting structural components. The working principle of an OPM relies on laser pumping of alkali metal atoms. OPMs can be classified into magnetically modulated systems and non-magnetically modulated systems based on the presence or absence of magnetic modulation, and can also be categorized as single-axis, dual-axis and triaxial OPM detectors according to the detectable magnetic field axes. Different detection systems require corresponding OPM control systems to precisely control these components.

Furthermore, magnetic shielding apparatuses are typically classified into two types: magnetic shielding cylinders and magnetic shielding rooms. For an OPM-based MEG system provided with the magnetic shielding cylinder, the subject lies on an acquisition bed, and after the OPM detector array is fixed, both are moved together to the bottom of the magnetic shielding cylinder. For an OPM-MEG system provided with the magnetic shielding room, the subject, after having the detector array fixed, performs MEG measurements inside the magnetic shielding room while staying stationary or making minor movements. Since OPMs are highly sensitive to direct current (DC) residual magnetism and alternating current (AC) magnetic noise, whereas OPM control systems typically generate residual magnetism and magnetic noise, the control system must be placed outside the magnetic shielding apparatuses to avoid interference. Under such circumstances, conventional OPM-MEG systems suffer from the following drawbacks:
1. For the OPM-MEG systems provided with the magnetic shielding cylinders, cables connecting OPMs and electronic systems generally need to be longer than 4 meters, resulting in extremely inconvenient operation. In addition, each MEG experiment requires repeated movement of these cables, which therefore must simultaneously possess low impedance, high shielding effectiveness, high mechanical strength, and high stability - all of which increase the cable's size, cost, and design difficulty.
2. The OPM-MEG systems provided with the magnetic shielding rooms also face cable-related challenges. Since electronic systems have to be placed outside the magnetic shielding apparatuses, the cable length typically needs to be over 4 meters. Meanwhile, the cable restricts the subject's movement during MEG testing.

Therefore, addressing the issues in existing OPM-MEG technology - such as stringent cable specifications, cumbersome data acquisition procedures, and restricted subject movement that limits paradigm design - to improve MEG data acquisition has become an urgent technical challenge that requires resolution.

### SUMMARY

The present disclosure aims to provide a wireless transmission-based optically pumped magnetometer-magnetoencephalography (OPM-MEG) system and an MEG detection method thereof, so as to improve the data acquisition performance of MEG.

The present disclosure may employ the following technical solutions.

The present disclosure provides a wireless transmission-based optically pumped magnetometer-magnetoencephalography (OPM-MEG) system, including a wearable structural member, an OPM, an acquisition host computer, and a control module. The wearable structural member is disposed in a magnetically shielded space. The OPM is disposed on the wearable structural member and configured to form a magnetic field detection array for detecting MEG signals. The acquisition host computer is disposed outside the magnetically shielded space and configured to store the MEG signals acquired by the OPM. The control module is disposed on the wearable structural member and electrically connected to the OPM via a flexible printed circuit. The control module is connected to the acquisition host computer via wireless communication. The control module is configured to perform state control and data transmission for the OPM in an uplink direction, receive wireless control commands and trigger instructions from the acquisition host computer in a downlink direction, and wirelessly transmit the acquired MEG signals to the acquisition host computer.

The present disclosure provides a magnetoencephalography (MEG) detection method, which adopts the aforementioned wireless transmission-based OPM-MEG system, the MEG detection method specifically including the steps of: disposing a wearable structural member in a magnetically shielded space and fitting the wearable structural member on a head of a subject; controlling, by a control module, an OPM to form a magnetic field detection array, and measuring MEG data of the subject using the magnetic field detection array; and transmitting, by a control module, the acquired MEG data to the acquisition host computer through wireless communication.

The present disclosure achieves the following advantageous effects.

When the wireless transmission-based OPM-MEG system is in use, the wearable structural member is fitted on the head of a subject, the OPM can form a magnetic field detection array for measuring MEG of the subject. Moreover, during use, the front end of the OPM can be secured to the scalp of the subject, which helps improve the accuracy of MEG measurements taken by the OPM and yields better results.

In addition, disposing the wearable structural member, the OPM, and the control module within the magnetically shielded space helps improve the measurement accuracy of the OPM. Meanwhile, since the control module is connected to the acquisition host computer via wireless communication, eliminating the need for a wired connection between the two. Thus, the subject is not constrained by wired cables during testing, which significantly improves the flexibility of the system and the freedom of movement of the subject, thereby addressing the problem in the prior art that paradigm design is limited due to restricted movement of the subject.

Further, by connecting the control module to the acquisition host computer via wireless communication, there is no need for wired circuits between them, nor is it necessary to consider specifications and costs of such circuits, thereby significantly reducing testing costs. Moreover, since the control module and the acquisition host computer do not use wired circuits to transmit signals, this helps reduce the magnetic noise generated by wired circuits during operation, thereby further improving the measurement accuracy of the OPM.

### BRIEF DESCRIPTION OF THE DRAWINGS

The accompanying drawings are merely intended to schematically illustrate and explain the present disclosure, rather than limiting the scope thereof.
FIG. 1 is a structural schematic diagram of a wireless transmission-based optically pumped magnetometer-magnetoencephalography (OPM-MEG) system according to the present disclosure;
FIG. 2 is a functional connection block diagram of a control module according to the present disclosure;
FIG. 3 is a structural schematic diagram of a single-axis OPM detector with dual-light non-magnetic modulation according to the present disclosure;
FIG. 4 is a structural schematic diagram of a dual-axis OPM detector with single-light magnetic modulation according to the present disclosure;
FIG. 5 is a structural schematic diagram of a triple-axis OPM detector with dual-light magnetic modulation according to the present disclosure;
FIG. 6 is a data plot measured in an empty room using the present disclosure;
FIG. 7 is a power spectrum plot of MEG signals acquired along an axis perpendicular to an occipital lobe using the present disclosure;
FIG. 8 is a time-domain waveform plot of the MEG signals shown in FIG. 7 after 7 Hz-13 Hz band-pass filtering;
FIG. 9 is a structural schematic diagram of the present disclosure applied in a magnetic shielding cylinder; and
FIG. 10 is a structural schematic diagram of the present disclosure applied in a magnetic shielding room.

### Reference numerals:

100: wearable structural member; 110: connecting frame;
200: OPM; 210: alkali metal atomic vapor cell; 220: laser;
230: magnetic compensation self-calibration modulation coil;
240: vapor cell heating temperature control module; 250: photodiode;
260: circular polarizer;
300: acquisition host computer;
400: control module; 410: main controller; 420: wireless communication module;
430: battery module; 431: non-magnetic battery pack;
432: non-magnetic battery drive module; 440: non-magnetic control circuit module;
450: non-magnetic acquisition circuit module;
500: wireless synchronizer; 600: stimulation host computer; 700: flexible printed circuit.

### DETAILED DESCRIPTION OF THE EMBODIMENTS

For a clearer understanding of the technical features, objectives and effects of the present disclosure, specific embodiments of the present disclosure will now be described with reference to the accompanying drawings.

### Embodiment 1

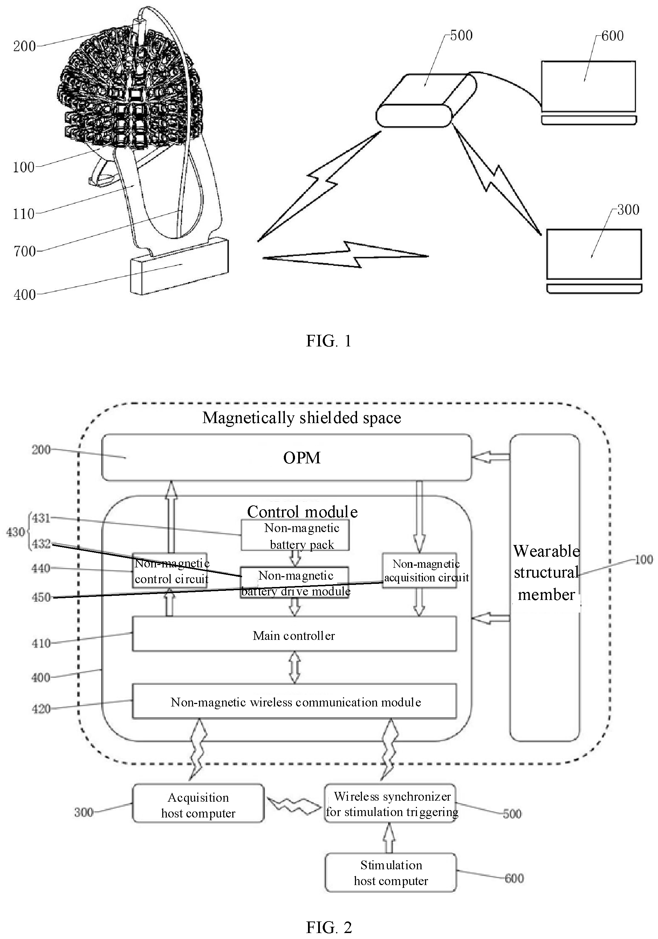

With reference to FIG. 1 and FIG. 2, embodiments of the present disclosure provide a wireless transmission-based optically pumped magnetometer-magnetoencephalography (OPM-MEG) system, including a wearable structural member 100 disposed in a magnetically shielded space, an optically pumped magnetometer (OPM) 200, an acquisition host computer 300, and a control module 400. The OPM 200 is disposed on the wearable structural member 100 and configured to form a magnetic field detection array for detecting magnetoencephalography (MEG) signals. The acquisition host computer 300 is disposed outside the magnetically shielded space and configured to store the MEG signals acquired by the OPM 200. The control module 400 is disposed on the wearable structural member 100 and electrically connected to the OPM 200 via a flexible printed circuit 700. The control module 400 is connected to the acquisition host computer 300 via wireless communication. The control module 400 is configured to: perform state control and data transmission for the OPM 200 in an uplink direction, receive wireless control commands and trigger instructions from the acquisition host computer in a downlink direction, and wirelessly transmit the acquired MEG signals to the acquisition host computer 300.

Overall, when the wireless transmission-based OPM-MEG system is in use, the wearable structural member 100 is fitted on the head of a subject, the OPM 200 can form a magnetic field detection array for measuring MEG of the subject. Moreover, during use, the front end of the OPM 200 can be secured to the scalp of the subject, which helps improve the accuracy of MEG measurements taken by the OPM 200 and yields better results.

In addition, disposing the wearable structural member 100, the OPM 200, and the control module 400 within the magnetically shielded space helps improve the measurement accuracy of the OPM 200. Meanwhile, since the control module 400 is connected to the acquisition host computer 300 via wireless communication, eliminating the need for a wired connection between the two. Thus, the subject is not constrained by wired cables during testing, which significantly improves the flexibility of the system and the freedom of movement of the subject, thereby addressing the problem in the prior art that paradigm design is limited due to restricted movement of the subject.

Further, by connecting the control module 400 to the acquisition host computer 300 via wireless communication, there is no need for wired circuits between them, nor is it necessary to consider specifications and costs of such circuits, thereby significantly reducing testing costs. Moreover, since the control module 400 and the acquisition host computer 300 do not use wired circuits to transmit signals, this helps reduce the magnetic noise generated by wired circuits during operation, thereby further improving the measurement accuracy of the OPM 200.

Designers can adjust the specific forming mode and size of the magnetically shielded space according to application requirements. For example, the magnetically shielded space may be formed by a shielding cylinder or a shielding room, which is not specifically limited herein.

In the present disclosure, through the cooperation between the control module 400 and the acquisition host computer 300, the acquired MEG data can be displayed in real time on the acquisition host computer 300 and stored remotely, similar to wired communication. Designers may determine the specific configurations of the acquisition host computer 300 and a stimulation host computer 600 according to application requirements - for example, a computer or controller loaded with corresponding programs - and no specific limitation is imposed herein.

Experimental research in the field of neuroscience often requires accurate recording of trigger timestamps in experimental paradigms and require trigger signals with synchronization functionality. In the prior art, wired transmission can effectively suppress delay jitter during signal transmission and accurately mark trigger timestamps. Nevertheless, when wireless communication is employed, how to better suppress delay jitter during signal transmission and accurately mark trigger timestamps has become a technical problem that urgently needs to be solved.

To solve the above problem, the embodiments of the present disclosure further include a clock synchronization module, which is communicatively connected to the control module 400 and the acquisition host computer 300 and configured to perform clock synchronization between the control module 400 and the acquisition host computer 300.

By synchronizing the clocks of the control module 400 and the acquisition host computer 300 through the clock synchronization module, clock synchronization between the two is ensured, thereby enabling better suppress of delay jitter during signal transmission and accurate marking of the trigger timestamps.

In a feasible embodiment, the clock synchronization module is disposed outside the magnetically shielded space. The clock synchronization module includes a wireless synchronizer 500 and a stimulation host computer 600. The wireless synchronizer 500 is connected to the control module 400 and the acquisition host computer 300 via wireless communication. The stimulation host computer 600 is communicatively connected to the wireless synchronizer 500 and is configured to transmit control signals for stimulation to the wireless synchronizer 500 for stimulation triggering. The wireless synchronizer 500 is configured to perform clock synchronization between the control module 400, the acquisition host computer 300, and the stimulation host computer 600.

The wireless synchronizer 500 can adopt a low-latency wireless communication protocol to synchronize magnetic field data and stimulation trigger signals.

By arranging the clock synchronization module outside the magnetically shielded space, the magnetic noise generated by the clock synchronization module is prevented from interfering the OPM 200.

In another feasible embodiment, the stimulation and control circuit modules may each adopt a high-precision synchronized crystal oscillator for independent timing, with synchronization performed only once at the start. Specifically, the stimulation and control circuit modules are disposed on the control module 400 and the acquisition host computer 300. Before use, the wireless synchronizer 500 performs one-time clock synchronization on the control module 400, the acquisition host computer 300, and the stimulation host computer 600.

For example, if the deviation between the crystal oscillators is 1 ppm, the time error between the stimulation trigger signal and the magnetic field data will not exceed 1 ms within 16.67 minutes without the need for a second synchronization operation.

In the prior art, the emission, transmission and reception of wireless electromagnetic waves occupy a wide frequency band. This includes not only electromagnetic wave variations caused by coding rates at the MHz and GHz frequency levels, but also transmission and reception packet responses within the frequency band of interest for MEG signals (1-100 Hz). Additionally, some wireless communication modes employ a heartbeat packet mechanism to maintain connections between devices. All of these factors will interfere with the detection of MEG signals by the OPM-MEG.

To solve the above problem, in the embodiments of the present disclosure, the wireless communication employs a packet transmission and reception time of no more than 1 ms, with the heartbeat packet protocol disabled.

Since the bandwidth of the OPM 200 is generally within the kHz frequency band, by modifying the communication protocol to make the packet transmission and reception time not greater than 1 ms and disabling interference protocols such as heartbeat packets, the noise caused by wireless communication is shifted out of the effective detection signal frequency band, thereby eliminating interference to the MEG signal acquisition at the communication protocol level.

Further, the transmit power of the wireless communication is controlled to be less than 0 dBm. The power control helps reduce spurious noise from the wireless communication signals.

Specifically, the wireless communication adopts one selected from the group consisting of a Bluetooth module, a Wi-Fi module, a LoRa module, and a Zigbee module. The specific communication protocol and transmit power may be determined and adjusted based on the selected communication module, which are not specifically limited herein.

Alternatively, in other feasible embodiments, designers may use other types of wireless communication modules 420 based on application requirements, which are not specifically limited herein.

In the embodiments of the present disclosure, the wearable structural member 100 includes a helmet and a connecting frame 110 connected to the helmet. The OPM 200 is disposed on the helmet, and the control module 400 is disposed on the connecting frame 110.

Mounting and securing the OPM 200 on the helmet provides enhanced stability, enabling the OPM 200 to form a magnetic field detection array on the helmet. This magnetic field detection array can be used to perform MEG measurement on different regions of the wearer's brain, which facilitates acquiring magnetoencephalograms with full-brain coverage.

Exemplarily, the helmet may be configured as a rigid structure. Configuring the helmet as a rigid structure can avoid deformation of the helmet during use and maintain its fixed shape, thereby helping sustain the stability of the OPM 200. The specific shape and size of the helmet 1 may be determined according to application requirements, which are not specifically limited herein.

Further, the connecting frame 110 is further connected to the helmet. Mounting the control module 400 on the connecting frame 110 not only facilitates the secure installation and fixation of the control module 400 but also helps reduce the distance between the OPM 200 and the control module 400, thereby shortening the length of the flexible printed circuit 700 between the OPM 200 and the control module 400.

Further, to prevent the control module 400 from being exposed, a protective housing may be disposed on the control module 400, and the control module 400 is connected to the connecting frame 110 through the protective housing. Designers may adjust the specific configuration of the connecting frame 110 according to application requirements, which is not specifically limited herein.

Alternatively, in other feasible embodiments, designers may adjust the specific configuration of the wearable structural member 100 so that the OPM 200 can be secured around the heart, thereby meeting the requirements for MCG measurement, which is not specifically limited herein.

In the embodiments of the present disclosure, the distance between the control module 400 and the OPM 200 is no less than 20 cm. Securing the control module 400 and the OPM 200 at a distance of more than 20 cm from each other helps reduce the interference from the control module 400 on the OPM 200. In addition, since the control module 400 can communicate wirelessly with the acquisition host computer 300, the use of wired circuits is significantly reduced, thereby lowering experimental costs.

In the prior art, disposing the OPM control circuit in the magnetically shielded space results in DC residual magnetism and AC magnetic noise generated by the circuit, both of which have a significant impact on detection results. These noise sources are likely to increase the local noise level of the environmental while introducing specific interference, severely degrading signal purity and signal-to-noise ratio, and consequently affecting the quality of the acquired data.

To solve the above problem, in the embodiments of the present disclosure, the control module 400 includes a main controller 410, a wireless communication module 420 disposed on the main controller 410, a battery module 430, a non-magnetic control circuit module 440, and a non-magnetic acquisition circuit module 450. The main controller 410 is electrically connected to the OPM 200 via the flexible printed circuit 700. At least one selected from the group consisting of the main controller 410, the wireless communication module 420, the battery module 430, the non-magnetic control circuit module 440, and the non-magnetic acquisition circuit module 450 is configured to be non-magnetic.

By configuring at least one selected from the group consisting of the main controller 410, the wireless communication module 420, the battery module 430, the non-magnetic control circuit module 440, and the non-magnetic acquisition circuit module 450 to be non-magnetic, generation of magnetic noise can be reduced, and the MEG measurement quality is improved. Exemplarily, the main controller 410, the wireless communication module 420, the non-magnetic battery module 430, the non-magnetic control circuit module 440, and the non-magnetic acquisition circuit module 450 are all configured to be non-magnetic.

In the embodiments of the present disclosure, the main controller 410 is configured to be non-magnetic. The main controller 410 includes a circuit board and electronic components disposed on the circuit board. The circuit board is printed via a non-magnetic process, and the electronic components are formed of non-magnetic materials.

By printing the circuit board via the non-magnetic process and forming the electronic components with non-magnetic materials, non-magnetization modification of the circuit board and the electronic components is achieved, thereby eliminating residual magnetic fields on the circuit board and the electronic components.

The specific preparation process is as follows: First, prepare a printed circuit board (PCB) via a non-magnetic manufacturing process. Second, in the circuit's schematic design, ferromagnetic electronic components (e.g., iron, nickel, cobalt, etc.) are not used; instead, components made of other materials such as ceramics are used for replacement, and the use of high-conductivity materials is limited to reduce magnetic noise. Finally, the components are soldered using solder that does not contain ferromagnetic materials.

The specific composition and configuration of the circuit board and the electronic components may be adjusted by designers according to application requirements, which are not specifically limited herein.

In the embodiments of the present disclosure, at least one selected from the non-magnetic control circuit module 440 and the non-magnetic acquisition circuit module 450 is configured to be non-magnetic. The non-magnetic control circuit module 440 and the non-magnetic acquisition circuit module 450 are electrically connected to the main controller 410, and are electrically connected to the OPM 200 via the flexible printed circuit 700. Exemplarily, both the non-magnetic control circuit module 440 and the non-magnetic acquisition circuit module 450 are configured to be non-magnetic.

The control module 400 can perform state control and data transmission of the OPM 200 in an uplink direction through the non-magnetic control circuit module 440, and the control module 400 can wirelessly transmit the acquired MEG signals downward to the acquisition host computer 300 in a downlink direction through the non-magnetic acquisition circuit module 450.

Specifically, at least one selected from the group consisting of the main controller 410, the non-magnetic control circuit module 440, and the non-magnetic acquisition circuit module 450 is each provided with a magnetic shielding layer, which is formed of a soft magnetic material to provide low-frequency magnetic field shielding for the at least one module selected from the group.

The specific type and composition of the soft magnetic material may be determined by designers according to application requirements (e.g., a soft magnetic alloy may be adopted), which are not specifically limited herein.

By demagnetizing the magnetic shielding layer and placing it in the magnetically shielded space, interference from residual magnetism and magnetic noise is minimized, thereby solving the problem of interferences of residual magnetism and magnetic noise caused by introducing the circuit into the magnetically shielded space.

The shielding layer is not disposed on the antenna used for wireless communication. In addition, the soft magnetic alloy shielding surrounding each circuit shall not be exposed to magnetic field environments such as geomagnetic fields, and should undergo demagnetization before each use.

Designers may adjust the specific position of the magnetic shielding layer according to application requirements, no specific limitations are imposed herein. Exemplarily, the main controller 410, the non-magnetic control circuit module 440, and the non-magnetic acquisition circuit module 450 are all provided with the magnetic shielding layer.

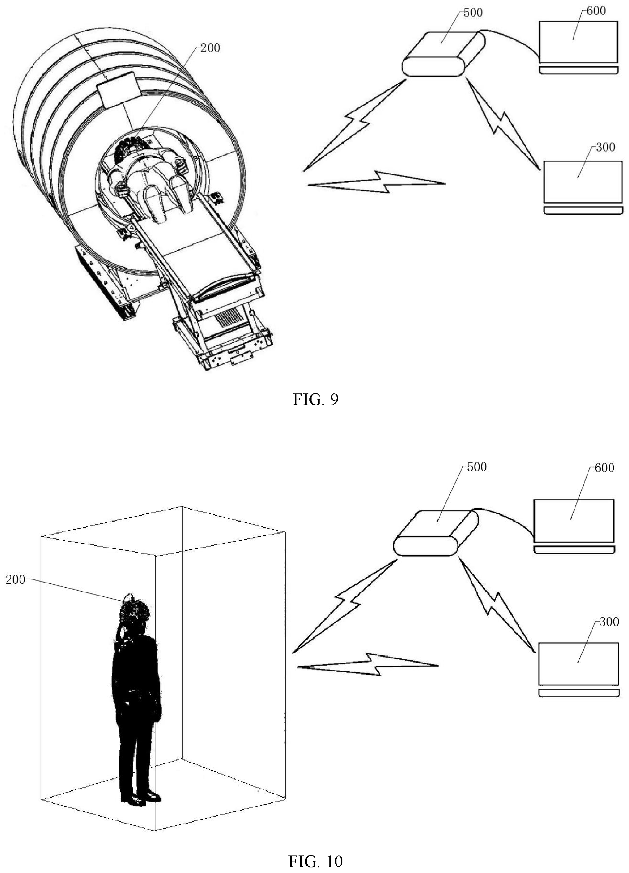

In the embodiments of the present disclosure, except for the battery module 430, the control module 400 is configured in a chip form via system-in-package packaging. Configuring the control module 400 in a chip form significantly reduces the volume of the control module 400, helps lower space occupation and corresponding power consumption, and renders the whole system more lightweight. The lightweight system can be freely placed inside the magnetically shielded space such as the magnetic shielding cylinder or the shielding room, as illustrated in the embodiments of FIG. 9 and FIG. 10, so as to better meet the wearable requirements of the MEG detection device.

In the prior art, the OPM-MEG is generally powered by a power grid. To realize wireless and portable OPM-MEG, batteries need to be integrated into the OPM control system to support independent operation of the OPM-MEG. However, after high-intensity discharge, the ferromagnetic metal materials inside conventional batteries retain magnetism to a certain extent. On one hand, the resulting residual magnetism creates local magnetic field inhomogeneity in the batteries. On the other hand, changes in the internal current also lead to the generation of magnetic noise.

To solve the above problems, in embodiments of the present disclosure, the battery module 430 is configured to be non-magnetic. The battery module 430 includes a non-magnetic battery pack 431 and a non-magnetic battery drive module 432. The non-magnetic battery pack 431 is formed of a non-magnetic lithium-ion polymer material, and the non-magnetic battery drive module 432 includes a non-magnetic boost voltage regulator circuit. The non-magnetic battery pack 431 is electrically connected to the main controller 410 via the non-magnetic boost voltage regulator circuit.

Using non-magnetic lithium-ion polymer batteries for power supply eliminates magnetic noise generated by ferromagnetic materials - such as nickel foil - used in most conventional lithium-ion batteries. Since the non-magnetic lithium-ion polymer batteries contain no ferromagnetic materials, they are better suited for environments where weak MEG signals are measured.

In addition, the non-magnetic battery pack 431 is electrically connected to the non-magnetic main controller 410 via the non-magnetic boost voltage regulator circuit. The non-magnetic battery pack 431 cooperates with the non-magnetic boost voltage regulator circuit to provide stable power support. Moreover, the non-magnetic boost voltage regulator circuit can prevent noise from being introduced into the detected MEG signals due to battery power variation. The non-magnetic boost voltage regulator circuit can be configured to be non-magnetic by means of a magnetic shielding layer. The specific configuration of the non-magnetic boost voltage regulator circuit may be determined by designers according to application requirements, which is not specifically limited herein.

In a feasible embodiment of the present disclosure, the wireless communication adopts an omnidirectional antenna, which can realize omnidirectional signal transceiving, thereby reducing directional design constraints on experimental paradigms.

In another feasible embodiment of the present disclosure, the wireless communication adopts a directional gain antenna. Based on the relative position of each component, the directional gain antenna performs high-transmission-power communication in the gain direction, which can extend the communication distance and reduce the interference of stray wireless communication signals on MEG detection.

In the embodiments of the present disclosure, the OPM 200 is any one selected from the group consisting of a non-magnetically modulated single-axis OPM detector, a magnetically modulated dual-axis OPM detector, and a magnetically modulated triple-axis OPM detector.

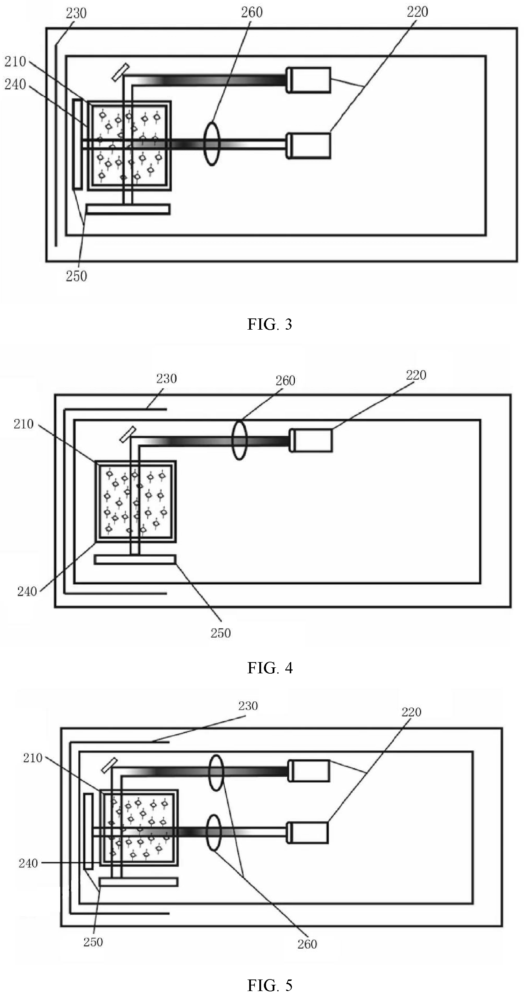

Specifically, in the embodiments illustrated in FIGS. 3 to 5, each of the non-magnetically modulated single-axis OPM detector, the magnetically modulated dual-axis OPM detector, and the magnetically modulated triple-axis OPM detector includes an alkali metal atomic vapor cell 210, a laser 220, a magnetic compensation self-calibration modulation coil 230, a vapor cell heating temperature control module 240, a photodiode 250, and a circular polarizer 260.

For the non-magnetically modulated OPM, pump light and probe light are perpendicular to each other and overlap at the alkali metal atomic vapor cell 210, and the magnetic field measurement direction is perpendicular to the two optical paths. For the magnetically modulated dual-axis measurement configuration, the direction of the modulation magnetic field is perpendicular to the pump light inside the alkali metal vapor cell. For the magnetically modulated triple-axis measurement configuration, two mutually perpendicular optical paths are formed inside the alkali metal vapor cell, and the two optical paths may be partially overlapped or completely non-overlapped.

In this system, the alkali metal atomic vapor cell 210 in the OPM 200 serves as the core component, and it is heated to a predetermined temperature by the vapor cell heating temperature control module 240 to vaporize alkali metal atoms. The alkali metal atoms stored in the vapor cell are polarized by pump light passing through the circular polarizer 260. The magnetic compensation self-calibration modulation coil 230 is configured to provide magnetic field modulation signals, magnetic field calibration, and magnetic field compensation. Magnetic field modulation enables measurement of multi-axis MEG signals; magnetic field calibration is used to calibrate the measured MEG signals in standard units; and the compensation magnetic field is used to further reduce the magnitude of the static magnetic field inside the vapor cell within the magnetic shielding environment. The photodiode 250 converts the acquired optical signals into electrical signals and transmits the electrical signals to the control module 400.

In this system, the control module 400 is mainly responsible for control and information transmission of the OPM 200. The control module 400 may include a main controller 410, a wireless communication module 420, a non-magnetic battery pack 431, a non-magnetic battery drive module 432, a non-magnetic control circuit module 440, and a non-magnetic acquisition circuit module 450.

In this system, the non-magnetic control circuit module 440 of the control module 400 is connected to the OPM 200 to control the OPM 200, and different control circuits correspond to different types of the OPM 200 respectively. Its main function is to generate control signals for temperature rise, frequency stabilization, atomic number density locking, magnetic field modulation, calibration, magnetic field compensation and the like for the OPM 200 according to control instructions from the main controller 410.

In this system, the non-magnetic acquisition circuit module 450 of the control module 400 is also connected to the OPM 200, and is configured to receive and amplify signals acquired by a probe and transmit the signals to the main controller 410.

In this system, the non-magnetic battery drive module 432 of the control module 400 is responsible for boosting the voltage of the non-magnetic battery pack 431 to the voltage required by the system, and is provided with a voltage regulation module to ensure that the power supply voltage to the control module 400 remains constant regardless of changes in battery charge.

In this system, the wireless communication module 420 of the control module 400 is responsible for transmitting instructions from the acquisition host computer 300 and trigger signals from the wireless synchronizer 500 for stimulation triggering to the main controller 410, and also transmits MEG acquisition signals from the main controller 410 to the acquisition host computer 300 in real time.

There are two schemes for synchronization design of the trigger signals:
1. Upon receiving a stimulation trigger signal from the stimulation host computer 600, the wireless synchronizer 500 for stimulation triggering adds its timestamp to the signal and sends the signal to the acquisition host computer 300.
2. Upon receiving a trigger signal received from the stimulation host computer 600, the wireless synchronizer 500 for stimulation triggering sends the signal directly to the acquisition host computer 300 via real-time wireless communication without adding a timestamp; meanwhile, the wireless communication module 420 also transmits MEG data received from the main controller 410 to the acquisition host computer 300 via real-time wireless communication through a different channel. The acquisition host computer 300 aligns the acquisition channel and the stimulation trigger channel based on its own clock.

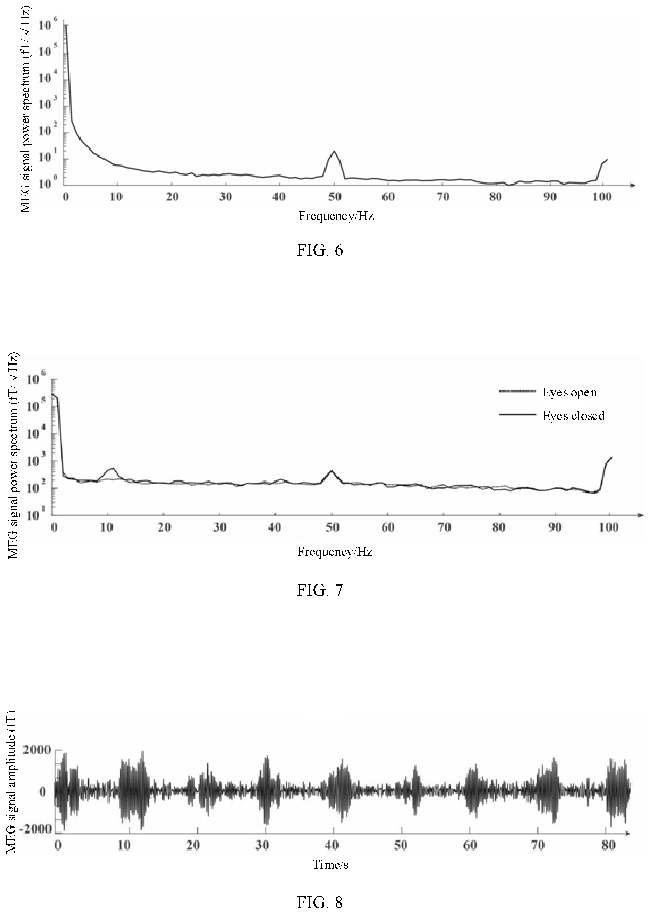

In the empty-room experiment, this system is placed in a region of uniform magnetic field within the magnetically shielded space, with no other objects placed except for the system of the present disclosure. Empty-room data is acquired for 30 seconds, and the power spectrum of the acquired signals is shown in FIG. 6.

In the eyes-open/eyes-closed alpha-wave (α-wave) suppression experiment, the OPM 200 is placed over the subject's occipital lobe at a distance of 2 cm from the midline fissure. This position is predetermined using a wired MEG acquisition system and verified as a channel exhibiting the strongest α-wave response among 30 occipital channels. The experimental paradigm consists of block-based cycles in which subjects close their eyes for 5 seconds and open their eyes for 5 seconds according to voice prompts, repeated 30 times. The power spectrum of MEG signals acquired along the axis perpendicular to the occipital lobe is shown in FIG. 7, and the time-domain MEG signals subjected to a 7-13 Hz band-pass filter are shown in FIG. 8.

Based on the above innovative design and improvement, the present disclosure realizes portable optimization of the wireless communication-based MEG system, and overcomes difficulties of conventional wired and wireless transmissions, thereby enabling broader applications in psychological, medical and other fields, and providing more comprehensive and in-depth data support for neuroscience and medical research.

In addition, the present disclosure performs an empty-room test and an eyes-open/eyes-closed α-wave suppression experiment to verify the feasibility and effectiveness of the system. The power spectrum of empty-room signals is shown in FIG. 6. It can be seen that the sensitivity of the wireless transmission-based OPM-MEG system in the present disclosure is approximately $20 fT / \sqrt{Hz} \left(10-20Hz\right)$. The power spectra of MEG signals acquired from the subject in the α-wave suppression experiment with eyes open and closed is shown in FIG. 7. The time-domain waveforms of the MEG signals processed by a 7-13 Hz band-pass filter (which contains the α-wave frequency band) are shown in FIG. 8. As can be seen from FIGS. 7 and 8, the present disclosure can accurately measure α-wave suppression under the eyes-open condition and α-wave signals under the eyes-closed condition of human subjects, and the power spectra exhibit significant differences in the α-wave frequency band between the eyes-open and eyes-closed conditions.

### Embodiment 2

Embodiments of the present disclosure provide a MEG detection method, which adopts the wireless transmission-based OPM-MEG system according to Embodiment 1. The MEG detection method specifically includes the following steps:
Step 1000: disposing a wearable structural member 100 in a magnetically shielded space and fitting the wearable structural member 100 on a head of a subject;
Step 2000: controlling, by a control module 400, an OPM 200 to form a magnetic field detection array, and measuring MEG data of the subject using the magnetic field detection array; and
Step 3000: transmitting, by the control module 400, the acquired MEG data to the acquisition host computer 300 through wireless communication.

The specific structure, the operating principle, and the effects of the wireless transmission-based OPM-MEG system are the same as those described in Embodiment 1, and will not be repeated herein.

The MEG detection method adopts the wireless transmission-based OPM-MEG system according to Embodiment 1, so that the control module 400 can communicate with the acquisition host computer 300 without wired circuits. Therefore, the subject is free from the restraints of wired circuits during measurement, which significantly improves usage flexibility and subject mobility, and addresses the limitations on paradigm design caused by restricted subject movement in the prior art.

In addition, the wireless communication connection between the acquisition host computer 300 and the control module 400 eliminates the need for wired circuits therebetween, and it is unnecessary to consider specifications and costs of wired circuits, thereby significantly reducing measurement costs.

In the embodiment of the present disclosure, the wireless transmission-based OPM-MEG system further includes a clock synchronization module. Before the step of measuring the MEG data of the subject using the magnetic field detection array, the method further includes:
Step 4000: performing, the clock synchronization module, clock synchronization between the control module 400 and the acquisition host computer 300.

The clock synchronization between the control module 400 and the acquisition host computer 300 ensures clock consistency therebetween, so as to better suppress delay jitter during signal transmission and accurately mark trigger timestamps.

Those described above are merely illustrative embodiments of the present disclosure, and are not intended to limit the scope thereof. Any equivalent variation or modification made by those skilled in the art without departing from the concept and principles of the present disclosure shall fall within the protection scope of the present disclosure.

## Claims

1. A wireless transmission-based optically pumped magnetometer-magnetoencephalography (OPM-MEG) system, comprising:
a wearable structural member disposed in a magnetically shielded space;
an optically pumped magnetometer (OPM) disposed on the wearable structural member and configured to form a magnetic field detection array for detecting magnetoencephalography (MEG) signals;
an acquisition host computer disposed outside the magnetically shielded space and configured to store the MEG signals acquired by the OPM; and
a control module disposed on the wearable structural member and electrically connected to the OPM via a flexible printed circuit, wherein the control module is connected to the acquisition host computer via wireless communication, and the control module is configured, within the magnetically shielded space, to:
perform state control and data transmission for the OPM in an uplink direction;
receive wireless control commands and trigger instructions from the acquisition host computer in a downlink direction; and
wirelessly transmit the acquired MEG signals to the acquisition host computer.

2. The wireless transmission-based OPM-MEG system according to claim 1, further comprising a clock synchronization module, which is communicatively connected to the control module and the acquisition host computer, and is configured to perform clock synchronization between the control module and the acquisition host computer.

3. The wireless transmission-based OPM-MEG system according to claim 2, wherein the clock synchronization module is disposed outside the magnetically shielded space and comprises:
a wireless synchronizer connected to the control module and the acquisition host computer via wireless communication; and
a stimulation host computer communicatively connected to the wireless synchronizer and configured to transmit a control signal issued for stimulation to the wireless synchronizer for stimulation triggering; wherein the wireless synchronizer is configured to perform clock synchronization between the control module, the acquisition host computer, and the stimulation host computer.

4. The wireless transmission-based OPM-MEG system according to any one of claims 1 to 3, wherein the wireless communication employs a packet transmission and reception time of no greater than 1 ms, with a heartbeat packet protocol disabled; and/or
the wireless communication has a transmission power of less than 0 dBm.

5. The wireless transmission-based OPM-MEG system according to claim 4, wherein the wireless communication adopts one selected from the group consisting of a Bluetooth module, a Wi-Fi module, a LoRa module, and a Zigbee module.

6. The wireless transmission-based OPM-MEG system according to claim 1, wherein the wearable structural member comprises a helmet and a connecting frame connected to the helmet, the OPM is disposed on the helmet, and the control module is disposed on the connecting frame.

7. The wireless transmission-based OPM-MEG system according to claim 1, wherein the control module comprises a main controller, a non-magnetic control circuit module disposed on the main controller, a non-magnetic acquisition circuit module, a wireless communication module, and a battery module;
the main controller is electrically connected to the OPM via the flexible printed circuit; and
at least one selected from the group consisting of the main controller, the non-magnetic control circuit module, the non-magnetic acquisition circuit module, the wireless communication module, the battery module, and the flexible printed circuit is configured to be non-magnetic.

8. The wireless transmission-based OPM-MEG system according to claim 7, wherein the main controller is configured to be non-magnetic, the main controller comprises a circuit board and electronic components disposed on the circuit board, the circuit board is printed via a non-magnetic process, and the electronic components are formed of non-magnetic materials.

9. The wireless transmission-based OPM-MEG system according to claim 7, wherein at least one selected from the non-magnetic control circuit module and the non-magnetic acquisition circuit module is configured to be non-magnetic, and the non-magnetic control circuit module and the non-magnetic acquisition circuit module are electrically connected to the main controller and the OPM, respectively.

10. The wireless transmission-based OPM-MEG system according to claim 8 or 9, wherein at least one selected from the group consisting of the main controller, the non-magnetic control circuit module, and the non-magnetic acquisition circuit module is provided with a magnetic shielding layer formed of a soft magnetic material to provide magnetic shielding for said at least one module.

11. The wireless transmission-based OPM-MEG system according to claim 7, wherein except for the battery module, the control module is configured in a chip form via non-magnetic system-in-package packaging.

12. The wireless transmission-based OPM-MEG system according to claim 7, wherein the battery module comprises a non-magnetic battery pack and a non-magnetic battery drive module;
the non-magnetic battery pack is formed of a non-magnetic lithium-ion polymer material, the non-magnetic battery drive module comprises a non-magnetic boost voltage regulator circuit, and the non-magnetic battery pack is electrically connected to the main controller via the non-magnetic boost voltage regulator circuit.

13. The wireless transmission-based OPM-MEG system according to claim 1, wherein the OPM is any one selected from the group consisting of a non-magnetically modulated single-axis OPM detector, a magnetically modulated dual-axis OPM detector, and a magnetically modulated triple-axis OPM detector.

14. The wireless transmission-based OPM-MEG system according to claim 1 or 3, wherein the wireless communication adopts an omnidirectional antenna or a directional gain antenna.

15. The wireless transmission-based OPM-MEG system according to claim 1, wherein a distance between the control module and the OPM is no less than 20 cm.

16. A magnetoencephalography (MEG) detection method, which adopts the wireless transmission-based OPM-MEG system according to any one of claims 1 to 15, the MEG detection method comprising the steps of:
disposing the wearable structural member in the magnetically shielded space and fitting the wearable structural member on a head of a subject;
controlling, by the control module, the OPM to form the magnetic field detection array, and measuring MEG data of the subject using the magnetic field detection array; and
transmitting, by the control module, the acquired MEG data to the acquisition host computer through wireless communication.

17. The MEG detection method according to claim 16, wherein the wireless transmission-based OPM-MEG system further comprises a clock synchronization module; and
before the step of measuring the MEG data of the subject using the magnetic field detection array, the method further comprises the step of:
performing, by the clock synchronization module, clock synchronization between the control module and the acquisition host computer.
